(19) Europäisches Patentamt
European Patent Office
Office européen des brevets

(11) Publication number: 0 563 392 A1

# (12) EUROPEAN PATENT APPLICATION published in accordance with Art. 158(3) EPC

(21) Application number: **92920054.1**

(22) Date of filing: **22.09.92**

(86) International application number: **PCT/JP92/01206**

(87) International publication number: **WO 93/06074 (01.04.93 93/09)**

(51) Int. Cl.5: **C07C 43/23**, C07C 43/295, C07C 49/84, C07C 205/37, C07C 317/22, C07C 323/20, C07C 323/21, C09B 11/00, C09B 57/00, C09B 62/00, B41M 5/26

(30) Priority: **24.09.91 JP 270549/91**
**03.10.91 JP 281926/91**
**11.09.92 JP 269791/92**

(43) Date of publication of application: **06.10.93 Bulletin 93/40**

(84) Designated Contracting States: **CH DE FR GB IT LI**

(71) Applicant: **NIPPON SODA CO., LTD.**
**2-1, Ohtemachi 2-chome**
**Chiyoda-ku, Tokyo 100(JP)**

(72) Inventor: **SATO, Takehiro, 1352-5, Ninomiya**
**Ninomiya-machi**
**Naka-gun**
**Kanagawa 259-01(JP)**
Inventor: **KAERIYAMA, Minoru, 36-3, Yahagi**
**Odawara-shi**
**Kanagawa 250(JP)**
Inventor: **YOSHINAKA, Shinzhi, 17-3, Kakura**
**1-chome**
**Iwatsuki-shi**
**Saitama 339(JP)**
Inventor: **HIDAKA, Tomoya, 1-34-302, Ukima**
**3-chome**
**Kita-ku**
**Tokyo 115(JP)**
Inventor: **KATAYAMA, Eizhi, 1176-1, Hujisawa**
**Nakago-mura**
**Nakakubiki-gun**
**Niigata 949-23(JP)**
Inventor: **KINOSHITA, Kimiaki, 94, Huruichiba**
**3-chome**
**Kitamoto-shi**
**Saitama 364(JP)**
Inventor: **UCHIKAWA, Masaaki, 1-34-404,**
**Ukima 3-chome**
**Kita-ku**
**Tokyo 115(JP)**

(74) Representative: **von Hellfeld, Axel, Dr. Dipl.-Phys.**
**Wuesthoff & Wuesthoff**
**Patent- und Rechtsanwälte**
**Schweigerstrasse 2**
**D-81541 München (DE)**

## (54) 2-PROPANOL COMPOUND AND RECORDING MATERIAL PREPARED THEREFROM.

(57) A 2-propanol compound represented by general formula (I), which is used as a preservative for colored images in heat-sensitive recording material and particularly improved in plasticizer resistance, wherein Y represents a group of general formula (II) or (III); $R^1$ represents hydrogen or alkyl; $R^2$ represents alkyl or alkoxy; $R^3$, $R^4$, $R^5$ and $R^6$ represent each alkyl or alkenyl; $R^7$ and $R^8$ represent each hydrogen, lower alkenyl or aralkyl; Z represents $-SO_2-$ or -CO-; ring A represents benzene or naphthalene; and n, m, p, q and r represent each an integer of 0 or above.

EP 0 563 392 A1

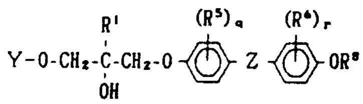 (I)

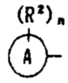 (II)

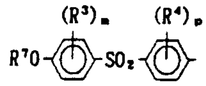 (III)

Technical Fields:

The invention is concerning to novel 2-propanol derivatives which act as stabilizing agent of colored image and to recording materials containing the compounds.

Background Art:

Recording materials applying reaction of leuco chromogens with color developers have been commonly used in thermal sensitive recording paper for facsimile, printer of various instruments to record output or presure sensitive multiple transcribing simultaneously slip books because the material can record only by relatively simple apparatus in a few moments without any complex procedure such as developing and fixing as photography.

The recording materials have been expected to be faster color development with clearer whiteness in uncolored area (Hereafter represented as "background") as well as higher toughness of the colored image and the background. Recently, the recording materials have been consumed increasingly in purposes that reliability of recorded image is critical such as package label. Accordingly the recording material with stability in storing colored image to plasticizers and fatty materials in organic macromolecules and fatty materials used for packaging has been intensively desired. Therefore, research efforts to solve the problems have been done in many approaches including research and development not only on various adjuvants, but also storage stabilizers or leuco chromogens and color developing materials. Nevertheless, any sufficient function has not been found.

Diphenylsulfone derivatives which are resemble to the compounds indicated by the general formula (I) in the present invention are pointed at first. They are well known as a color developer for recording materials. Diphenylsulfone derivatives substituted with alkoxy or aralkoxy at one side ring and hydroxylated at another side were proposed in Japanese Patent Application (Unexamined) Sho 57(1982)-210886 , ibid. Sho 58(1983)-20493, ibid. Sho 58(1983)-82788, ibid. Sho 58(1983)-132593, ibid. Sho 60(1985)-13852 and World Patent: World Open 84/02882 (unexamined).

On the other hand, color developers composed of 2-propanol derivatives in which p-hydroxyphenylthio and aryloxy groups as substituents were proposed in Japanese Patent Application (Unexamined) Hei 2-(1990)-145563 and ibid. Hei 2(1990)-167797, etc. The derivatives substituted by 1,3-diaryloxy group were proposed as a adjuvant in Japanese Patent Application (Unexamined) Hei 2(1990)-145535, etc. However, they are difficult to be acceptable with satisfaction in respect to the storing stability. The inventors also found that 4-benzyloxy-4'-(2-methylglycidyloxy) diphenylsulfone was effective as a storing image stabilizing agent. Nevertheless, it has been difficult to evaluate satisfactorily in respect of proof against plasticizer.

As described above, in recent year, improvement of the image storing stability of colored recording materials, and especially proofing effect against plasticizer have been expected. The purpose of the present invention is to provide a recording material with outstanding storage stability of colored image.

Disclosure of Invention:

The present invention is concerning to novel 2-propanol derivatives represented by general formula (I) and a recording material characterized by containing one or more the derivatives.

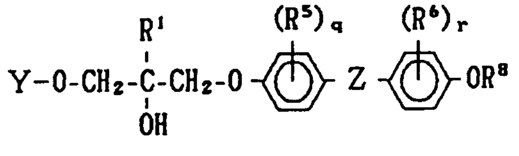 (I)

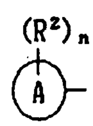 (II)

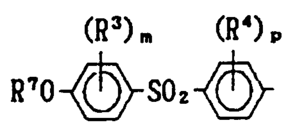 (III)

(Wherein: Y is a group represented by general formula (II) or (III)

$R^1$ is hydrogen or lower alkyl,

$R^2$ is halogen, nitro, lower alkyl, lower alkoxy, lower alkylthio, lower alkylsulfonyl, aryl, aryloxy, arylthio

or arylsulfonyl, in which aryl may be substituted,

each of $R^3$, $R^4$, $R^5$ and $R^6$ independently demonstrates halogen, lower alkyl, lower alkoxy, lower alkenyl or lower alkenyloxy,

each of $R^7$ and $R^8$ is independently hydrogen, lower alkyl, lower alkenyl or aralkyl,

Z is bivalent lower alkyl, $-SO_2-$, -CO-, -S- or -O-,

ring A is benzene or naphthalene ring,

n, m, p, q and r denote integral number from 0 to 4, however if n, m p, q or r is 2 or more, $R^2$, $R^3$, $R^4$, $R^5$ or $R^7$ may be different, respectively, in each substituent.)

Describing additionally in more detail on the substituents in the general formula (I), lower alkyl, lower alkoxy, lower alkenyl or lower alkenyloxy contains 1 to 5 carbons and may have side chain. Aralkyl indicates benzyl or phenethyl that may be substituted by halogen, nitro, lower alkyl or lower alkoxy at the benzene ring. "Aryl" of aryl, aryloxy, arylthio or arylsulfonyl group in the substituent at ring A that is benzene or naphthalene, may be substituted and the substituent may be halogen, nitro, lower alkyl or alkoxy. The alkyl and alkoxy, which may have a side chain, have 1 through 5 carbons.

Actual examples of 2-propanol derivatives represented by general formula (I) will be described below, however, the invention shall not be restricted in the examples.

The typical example of 2-propanol derivatives represented by general formula (I) in which Y is general formula (II) and (III), are shown in Table 1 and 2, respectively.

TABLE 1

general formula (II)

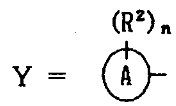

general formula (I)

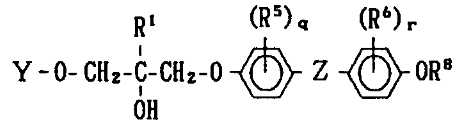

The mark " ——— " in the table shows hydrogen (no substituent).

| Compound No. | Ring A | $R^1$ | $(R^2)n$ | $(R^5)q$ | $(R^6)r$ | $R^8$ | Z | m.p. ℃ |
|---|---|---|---|---|---|---|---|---|
| 1 - 1 | benzene ring | H | ——— | ——— | ——— | H | $-SO_2-$ | 145-148 |
| 1 - 2 | benzene ring | H | ——— | $3-CH_3$ | $3-CH_3$ | H | $-SO_2-$ | 168-170 |
| 1 - 3 | benzene ring | H | ——— | $3,5-(CH_3)_2$ | $3,5-(CH_3)_2$ | H | $-SO_2-$ | 150-154 |
| 1 - 4 | benzene ring | H | ——— | $3,5-Br_2$ | $3,5-Br_2$ | H | $-SO_2-$ | oily |
| 1 - 5 | benzene ring | H | ——— | $3-CH_2CH=CH_2$ | $3-CH_2CH=CH_2$ | H | $-SO_2-$ | |
| 1 - 6 | benzene ring | H | ——— | ——— | ——— | $-CH_3$ | $-SO_2-$ | |
| 1 - 7 | benzene ring | H | ——— | ——— | ——— | $n-C_3H_7$ | $-SO_2-$ | |
| 1 - 8 | benzene ring | H | ——— | ——— | ——— | $i-C_3H_7$ | $-SO_2-$ | |
| 1 - 9 | benzene ring | H | ——— | ——— | ——— | $-CH_2C_6H_5$ | $-SO_2-$ | |
| 1 - 10 | benzene ring | H | $4-CH_3$ | ——— | ——— | H | $-SO_2-$ | 170-172 |
| 1 - 11 | benzene ring | H | $4-CH_3$ | $3-CH_2CH=CH_2$ | $3-CH_2CH=CH_2$ | H | $-SO_2-$ | 170-172 |
| 1 - 12 | benzene ring | H | $3-CH_3$ | ——— | ——— | H | $-SO_2-$ | 135-138 |
| 1 - 13 | benzene ring | H | $2-CH_3$ | ——— | ——— | H | $-SO_2-$ | 158-160 |
| 1 - 14 | benzene ring | H | 4-Cl | ——— | ——— | H | $-SO_2-$ | 162-163 |

TABLE 1 (continue)

| Compound No. | Ring A | $R^1$ | $(R^2)n$ | $(R^5)q$ | $(R^6)r$ | $R^8$ | Z | m. p. ℃ |
|---|---|---|---|---|---|---|---|---|
| 1 − 15 | benzene ring | H | 3-Cl | ——— | ——— | H | $-SO_2-$ | 119-120 |
| 1 − 16 | benzene ring | H | 2,4-$(CH_3)_2$ | ——— | ——— | H | $-SO_2-$ | 126-130 |
| 1 − 17 | benzene ring | H | 3,5-$(CH_3)_2$ | ——— | ——— | H | $-SO_2-$ | 153-156 |
| 1 − 18 | benzene ring | H | 4-$C_2H_5$ | ——— | ——— | H | $-SO_2-$ | 135-138 |
| 1 − 19 | benzene ring | H | 4-i-$C_3H_7$ | ——— | ——— | H | $-SO_2-$ | 56- 60 |
| 1 − 20 | benzene ring | H | 4-t-$C_4H_9$ | ——— | ——— | H | $-SO_2-$ | 132-135 |
| 1 − 21 | benzene ring | H | 4-$C_6H_5$ | ——— | ——— | H | $-SO_2-$ | 180-183 |
| 1 − 22 | benzene ring | H | 4-$OCH_3$ | ——— | ——— | H | $-SO_2-$ | 123-124 |
| 1 − 23 | benzene ring | H | 4-$NO_2$ | ——— | ——— | H | $-SO_2-$ | 139-145 |
| 1 − 24 | benzene ring | H | 2-t-$C_4H_9$<br>4-$CH_3$ | ——— | ——— | H | $-SO_2-$ | oily |
| 1 − 25 | benzene ring | H | 2,4,6-$(CH_3)_3$ | ——— | ——— | H | $-SO_2-$ | 196-198 |
| 1 − 26 | benzene ring | H | 4-O-$C_6H_5$ | ——— | ——— | H | $-SO_2-$ | |
| 1 − 27 | benzene ring | H | 3-O-$C_6H_5$ | ——— | ——— | H | $-SO_2-$ | |
| 1 − 28 | benzene ring | H | 4-S-$C_6H_5$ | ——— | ——— | H | $-SO_2-$ | |
| 1 − 29 | benzene ring | H | 4-$SO_2$-$CH_3$ | ——— | ——— | H | $-SO_2-$ | |
| 1 − 31 | benzene ring | H | 3-$SO_2$-$CH_3$ | ——— | ——— | H | $-SO_2-$ | |
| 1 − 32 | benzene ring | H | 4-$SO_2$-$C_6H_5$ | ——— | ——— | H | $-SO_2-$ | |
| 1 − 33 | benzene ring | H | 3-$SO_2$-$C_6H_5$ | ——— | ——— | H | $-SO_2-$ | |
| 1 − 34 | benzene ring | H | 4-S-$CH_3$ | ——— | ——— | H | $-SO_2-$ | |
| 1 − 35 | benzene ring | H | 3-S-$CH_3$ | ——— | ——— | H | $-SO_2-$ | |
| 1 − 36 | benzene ring | H | 3-S-$C_2H_5$ | ——— | ——— | H | $-SO_2-$ | |

TABLE 1 (continue)

| Compound No. | Ring A | $R^1$ | $(R^2)n$ | $(R^5)q$ | $(R^6)r$ | $R^8$ | Z | m.p. ℃ |
|---|---|---|---|---|---|---|---|---|
| 1 − 37 | benzene ring | H | $4-CH_2-C_6H_5$ | ——— | ——— | H | $-SO_2-$ | |
| 1 − 38 | benzene ring | H | $3-CH_2-C_6H_5$ | ——— | ——— | H | $-SO_2-$ | |
| 1 − 39 | benzene ring | H | $4-CO-C_6H_5$ | ——— | ——— | H | $-SO_2-$ | |
| 1 − 40 | benzene ring | H | $3-CO-C_6H_5$ | ——— | ——— | H | $-SO_2-$ | |
| 1 − 41 | 2-naphthalene ring | H | ——— | ——— | ——— | H | $-SO_2-$ | 184-186 |
| 1 − 42 | benzene ring | $-CH_3$ | ——— | ——— | ——— | H | $-SO_2-$ | 102-105 |
| 1 − 43 | benzene ring | $-CH_3$ | ——— | $3-CH_2CH=CH_2$ | $3-CH_2CH=CH_2$ | H | $-SO_2-$ | |
| 1 − 44 | benzene ring | $-CH_3$ | ——— | ——— | ——— | $-CH_3$ | $-SO_2-$ | |
| 1 − 45 | benzene ring | $-CH_3$ | ——— | ——— | ——— | $n-C_3H_7$ | $-SO_2-$ | |
| 1 − 46 | benzene ring | $-CH_3$ | ——— | ——— | ——— | $i-C_3H_7$ | $-SO_2-$ | |
| 1 − 47 | benzene ring | $-CH_3$ | ——— | ——— | ——— | $-CH_2C_6H_5$ | $-SO_2-$ | |
| 1 − 48 | benzene ring | $-CH_3$ | 4-Cl | ——— | ——— | H | $-SO_2-$ | 146-149 |
| 1 − 49 | benzene ring | $-CH_3$ | 3-Cl | ——— | ——— | H | $-SO_2-$ | 95-100 |
| 1 − 50 | benzene ring | $-CH_3$ | 2-Cl | ——— | ——— | H | $-SO_2-$ | oily |
| 1 − 51 | benzene ring | $-CH_3$ | $2,4-(CH_3)_2$ | ——— | ——— | H | $-SO_2-$ | oily |
| 1 − 52 | benzene ring | $-CH_3$ | $3,5-(CH_3)_2$ | ——— | ——— | H | $-SO_2-$ | 112-125 |
| 1 − 53 | benzene ring | $-CH_3$ | $4-C_2H_5$ | ——— | ——— | H | $-SO_2-$ | 98-103 |
| 1 − 54 | benzene ring | $-CH_3$ | $4-i-C_3H_7$ | ——— | ——— | H | $-SO_2-$ | 62- 66 |
| 1 − 55 | benzene ring | $-CH_3$ | $4-t-C_4H_9$ | ——— | ——— | H | $-SO_2-$ | 153-158 |
| 1 − 56 | benzene ring | $-CH_3$ | $4-C_6H_5$ | ——— | ——— | H | $-SO_2-$ | 163-165 |
| 1 − 57 | benzene ring | $-CH_3$ | $4-OCH_3$ | ——— | ——— | H | $-SO_2-$ | oily |
| 1 − 58 | benzene ring | $-CH_3$ | $4-NO_2$ | ——— | ——— | H | $-SO_2-$ | 122-128 |

TABLE 1 (continue)

| Compound No. | Ring A | $R^1$ | $(R^2)n$ | $(R^5)q$ | $(R^6)r$ | $R^8$ | Z | m.p. ℃ |
|---|---|---|---|---|---|---|---|---|
| 1 − 59 | benzene ring | $-CH_3$ | 2-t-$C_4H_9$ 4-$CH_3$ | ——— | ——— | H | $-SO_2-$ | 89- 93 |
| 1 − 60 | benzene ring | $-CH_3$ | 2,4,6-$(CH_3)_3$ | ——— | ——— | H | $-SO_2-$ | 131-136 |
| 1 − 61 | benzene ring | $-CH_3$ | 4-O-$C_6H_5$ | ——— | ——— | H | $-SO_2-$ | |
| 1 − 62 | benzene ring | $-CH_3$ | 4-$SO_2$-$CH_3$ | ——— | ——— | H | $-SO_2-$ | |
| 1 − 63 | benzene ring | $-CH_3$ | 4-S-$C_6H_5$ | ——— | ——— | H | $-SO_2-$ | |
| 1 − 64 | benzene ring | $-CH_3$ | 4-S-$CH_3$ | ——— | ——— | H | $-SO_2-$ | |
| 1 − 65 | 2-naphthalene ring | $-CH_3$ | ——— | ——— | ——— | H | $-SO_2-$ | 102-104 |
| 1 − 66 | benzene ring | H | ——— | ——— | ——— | H | $-C(CH_3)_2-$ | 128-131 |
| 1 − 67 | benzene ring | H | 4-$CH_3$ | ——— | ——— | H | $-C(CH_3)_2-$ | 116-118 |
| 1 − 68 | benzene ring | H | 4-$OCH_3$ | ——— | ——— | H | $-C(CH_3)_2-$ | 116-118 |
| 1 − 69 | benzene ring | H | 4-Cl | ——— | ——— | H | $-C(CH_3)_2-$ | 139-142 |
| 1 − 70 | benzene ring | $-CH_3$ | ——— | ——— | ——— | H | $-C(CH_3)_2-$ | 128-131 |
| 1 − 71 | benzene ring | $-CH_3$ | 4-$CH_3$ | ——— | ——— | H | $-C(CH_3)_2-$ | 116-118 |
| 1 − 72 | benzene ring | $-CH_3$ | 4-$OCH_3$ | ——— | ——— | H | $-C(CH_3)_2-$ | 116-118 |
| 1 − 73 | benzene ring | $-CH_3$ | 4-Cl | ——— | ——— | H | $-C(CH_3)_2-$ | 139-142 |
| 1 − 74 | benzene ring | H | ——— | 2-$CH_3$ | 2-$CH_3$ | H | $-C(CH_3)_2-$ | oily |
| 1 − 75 | benzene ring | H | ——— | 2,6-$Br_2$ | 2,6-$Br_2$ | H | $-C(CH_3)_2-$ | oily |
| 1 − 76 | benzene ring | H | ——— | ——— | ——— | H | $-CH(CH_3)-$ | 95-99 |
| 1 − 77 | benzene ring | H | ——— | ——— | ——— | H | $-(CH_3)C(C(CH_3)_3)-$ | oily |
| 1 − 78 | benzene ring | H | ——— | ——— | ——— | H | $-CO-$ | 133-134 |
| 1 − 79 | benzene ring | H | 4-$CH_3$ | ——— | ——— | H | $-CO-$ | 146-147 |

TABLE 1 (continue)

| Compound No. | Ring A | $R^1$ | $(R^2)n$ | $(R^5)q$ | $(R^6)r$ | $R^8$ | Z | m.p. ℃ |
|---|---|---|---|---|---|---|---|---|
| 1-80 | benzene ring | H | 4-Cl | — | — | H | -CO- | 132-133 |
| 1-81 | benzene ring | H | 2,4,6-$(CH_3)_3$ | — | — | H | -CO- | oily |
| 1-82 | benzene ring | H | 2-$CH_3$ | — | — | H | -CO- | 142-143 |
| 1-83 | benzene ring | H | 3-$CH_3$ | — | — | H | -CO- | 163-165 |
| 1-84 | benzene ring | H | 3,5-$(CH_3)_2$ | — | — | H | -CO- | 134-136 |
| 1-85 | benzene ring | -$CH_3$ | — | — | — | H | -CO- | 159-162 |
| 1-86 | benzene ring | -$CH_3$ | 4-$CH_3$ | — | — | H | -CO- | 186-187 |
| 1-87 | benzene ring | -$CH_3$ | 4-Cl | — | — | H | -CO- | 186-187 |
| 1-88 | benzene ring | -$CH_3$ | 4-t-$C_4H_9$ | — | — | H | -CO- | oily |
| 1-89 | 2-naphthalene ring | H | — | — | — | H | -CO- | 188-191 |
| 1-90 | benzene ring | H | — | — | — | H | -S- | 128-130 |
| 1-91 | benzene ring | H | 4-$CH_3$ | — | — | H | -S- | |
| 1-92 | benzene ring | H | 4-Cl | — | — | H | -S- | |
| 1-93 | benzene ring | -$CH_3$ | — | — | — | H | -S- | 128-130 |
| 1-94 | benzene ring | -$CH_3$ | 4-$CH_3$ | — | — | H | -S- | |
| 1-95 | benzene ring | -$CH_3$ | 4-Cl | — | — | H | -S- | |
| 1-96 | benzene ring | H | — | — | — | H | -O- | 90- 94 |
| 1-97 | benzene ring | H | 4-$CH_3$ | — | — | H | -O- | |
| 1-98 | benzene ring | H | 4-Cl | — | — | H | -O- | |
| 1-99 | benzene ring | -$CH_3$ | — | — | — | H | -O- | 90- 94 |
| 1-100 | benzene ring | -$CH_3$ | 4-$CH_3$ | — | — | H | -O- | |
| 1-101 | benzene ring | -$CH_3$ | 4-Cl | — | — | H | -O- | |

TABLE 2

general formula (III)

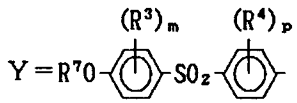

general formula (I)

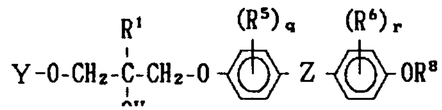

The mark " ——— " in the table shows hydrogen (no substituent).

| Compound No. | $R^1$ | $(R^3)m$ | $(R^4)p$ | $(R^5)q$ | $(R^6)r$ | $R^7$ | $R^8$ | Z | m.p. ℃ |
|---|---|---|---|---|---|---|---|---|---|
| 2 − 1 | H | ——— | ——— | ——— | ——— | $-CH_2C_6H_5$ | $-CH_2C_6H_5$ | $-SO_2-$ | 201-205 |
| 2 − 2 | H | ——— | ——— | ——— | ——— | $-i-C_3H_7$ | $-i-C_3H_7$ | $-SO_2-$ | 165-167 |
| 2 − 3 | H | ——— | ——— | ——— | ——— | $-CH_2C_6H_5$ | $-i-C_3H_7$ | $-SO_2-$ | 171-172 |
| 2 − 4 | H | ——— | ——— | ——— | ——— | $-CH_2CH_2C_6H_5$ | $-CH_2CH_2C_6H_5$ | $-SO_2-$ | |
| 2 − 5 | H | ——— | ——— | ——— | ——— | $-CH_2CH=CH_2$ | $-CH_2CH=CH_2$ | $-SO_2-$ | |
| 2 − 6 | H | ——— | ——— | ——— | ——— | $-CH_2C(CH_3)=CH_2$ | $-CH_2C(CH_3)=CH_2$ | $-SO_2-$ | |
| 2 − 7 | H | ——— | ——— | ——— | ——— | $-CH_2C(CH_3)=CH_2$ | $-CH_2CH_2C_6H_5$ | $-SO_2-$ | |
| 2 − 8 | H | ——— | ——— | ——— | ——— | $-i-C_3H_7$ | $-CH_2CH_2C_6H_5$ | $-SO_2-$ | |
| 2 − 9 | H | ——— | ——— | ——— | ——— | $-i-C_3H_7$ | $-CH_2C(CH_3)=CH_2$ | $-SO_2-$ | |
| 2 − 10 | H | ——— | ——— | ——— | ——— | $C_6H_5CH_2-$ | $-CH_2C(CH_3)=CH_2$ | $-SO_2-$ | |
| 2 − 11 | H | $3-CH_3$ | $3-CH_3$ | $3-CH_3$ | $3-CH_3$ | $-CH_2C_6H_5$ | $-CH_2C_6H_5$ | $-SO_2-$ | |
| 2 − 12 | H | $3-CH_3$ | $3-CH_3$ | ——— | ——— | $-CH_2C_6H_5$ | $-i-C_3H_7$ | $-SO_2-$ | |
| 2 − 13 | H | $3-CH_2CH=CH_2$ | $3-CH_2CH=CH_2$ | $3-CH_2CH=CH_2$ | $3-CH_2CH=CH_2$ | $-CH_2C_6H_5$ | $-CH_2C_6H_5$ | $-SO_2-$ | |
| 2 − 14 | H | $3,5-(CH_3)_2$ | $3,5-(CH_3)_2$ | $3,5-(CH_3)_2$ | $3,5-(CH_3)_2$ | $-CH_2C_6H_5$ | $-CH_2C_6H_5$ | $-SO_2-$ | |
| 2 − 15 | H | $3,5-Br_2$ | $3,5-Br_2$ | $3,5-Br_2$ | $3,5-Br_2$ | $-CH_2C_6H_5$ | $-CH_2C_6H_5$ | $-SO_2-$ | |

TABLE 2 (continue)

| Compound No. | $R^1$ | $(R^3)m$ | $(R^4)p$ | $(R^5)q$ | $(R^6)r$ | $R^7$ | $R^8$ | Z | m.p. ℃ |
|---|---|---|---|---|---|---|---|---|---|
| 2－16 | H | 3-$CH_2CH=CH_2$ | 3-$CH_2CH=CH_2$ | 3,5-$(CH_3)_2$ | 3,5-$(CH_3)_2$ | -$CH_2C_6H_5$ | -$CH_2C_6H_5$ | -$SO_2$- | |
| 2－17 | H | 3,5-$(CH_3)_2$ | 3,5-$(CH_3)_2$ | 3,5-$(CH_3)_2$ | 3,5-$(CH_3)_2$ | H | H | -$SO_2$- | |
| 2－18 | H | 3-$CH_2CH=CH_2$ | 3-$CH_2CH=CH_2$ | 3-$CH_2CH=CH_2$ | 3-$CH_2CH=CH_2$ | H | H | -$SO_2$- | |
| 2－19 | H | 3,5-$Br_2$ | 3,5-$Br_2$ | 3-$CH_2CH=CH_2$ | 3-$CH_2CH=CH_2$ | H | H | -$SO_2$- | |
| 2－20 | H | 3,5-$Br_2$ | 3,5-$Br_2$ | 3-$CH_3$ | 3-$CH_3$ | H | H | -$SO_2$- | |
| 2－21 | H | 3,5-$Br_2$ | 3,5-$Br_2$ | 3,5-$(CH_3)_2$ | 3,5-$(CH_3)_2$ | H | H | -$SO_2$- | |
| 2－22 | H | 3,5-$Br_2$ | 3,5-$Br_2$ | — | — | H | H | -$SO_2$- | |
| 2－23 | H | 3,5-$Br_2$ | 3,5-$Br_2$ | 3,5-$Br_2$ | 3,5-$Br_2$ | H | H | -$SO_2$- | |
| 2－24 | H | — | — | — | — | H | H | -$SO_2$- | 168-170 |
| 2－25 | -$CH_3$ | — | — | — | — | -$CH_2C_6H_5$ | -$CH_2C_6H_5$ | -$SO_2$- | 204-205 |
| 2－26 | -$CH_3$ | — | — | — | — | -i-$C_3H_7$ | -i-$C_3H_7$ | -$SO_2$- | 186-188 |
| 2－27 | -$CH_3$ | — | — | — | — | -$CH_2C_6H_5$ | -i-$C_3H_7$ | -$SO_2$- | 217-220 |
| 2－28 | -$CH_3$ | — | — | — | — | -$CH_2CH_2C_6H_5$ | -$CH_2CH_2C_6H_5$ | -$SO_2$- | |
| 2－29 | -$CH_3$ | — | — | — | — | -$CH_2CH=CH_2$ | -$CH_2CH=CH_2$ | -$SO_2$- | |
| 2－30 | -$CH_3$ | — | — | — | — | -$CH_2C(CH_3)=CH_2$ | -$CH_2C(CH_3)=CH_2$ | -$SO_2$- | |
| 2－31 | -$CH_3$ | — | — | — | — | -$CH_2C(CH_3)=CH_2$ | -$CH_2CH_2C_6H_5$ | -$SO_2$- | |
| 2－32 | -$CH_3$ | — | — | — | — | -i-$C_3H_7$ | -$CH_2CH_2C_6H_5$ | -$SO_2$- | |
| 2－33 | -$CH_3$ | — | — | — | — | -i-$C_3H_7$ | -$CH_2C(CH_3)=CH_2$ | -$SO_2$- | |
| 2－34 | -$CH_3$ | — | — | — | — | $C_6H_5CH_2$- | -$CH_2C(CH_3)=CH_2$ | -$SO_2$- | |
| 2－35 | -$CH_3$ | 3-$CH_3$ | 3-$CH_3$ | — | — | -$CH_2CH_2C_6H_5$ | -$CH_2CH_2C_6H_5$ | -$SO_2$- | |
| 2－36 | -$CH_3$ | 3-$CH_3$ | 3-$CH_3$ | — | — | -$CH_2C_6H_5$ | -i-$C_3H_7$ | -$SO_2$- | |
| 2－37 | -$CH_3$ | 3-$CH_2CH=CH_2$ | 3-$CH_2CH=CH_2$ | 3-$CH_2CH=CH_2$ | 3-$CH_2CH=CH_2$ | -$CH_2C_6H_5$ | -$CH_2C_6H_5$ | -$SO_2$- | |

TABLE 2 (continue)

| Compound No. | $R^1$ | $(R^3)m$ | $(R^4)p$ | $(R^5)q$ | $(R^6)r$ | $R^7$ | $R^8$ | Z | m. p. °C |
|---|---|---|---|---|---|---|---|---|---|
| 2-38 | $-CH_3$ | $3-CH_3$ | $3-CH_3$ | $3-CH_3$ | $3-CH_3$ | $-CH_2C_6H_5$ | $-CH_2C_6H_5$ | $-SO_2-$ | |
| 2-39 | $-CH_3$ | $3,5-(CH_3)_2$ | $3,5-(CH_3)_2$ | $3,5-(CH_3)_2$ | $3,5-(CH_3)_2$ | $-CH_2C_6H_5$ | $-CH_2C_6H_5$ | $-SO_2-$ | |
| 2-40 | $-CH_3$ | $3-CH_2CH=CH_2$ | $3-CH_2CH=CH_2$ | $3,5-(CH_3)_2$ | $3,5-(CH_3)_2$ | $-CH_2C_6H_5$ | $-CH_2C_6H_5$ | $-SO_2-$ | |
| 2-41 | $-CH_3$ | $3,5-Br_2$ | $3,5-Br_2$ | $3,5-Br_2$ | $3,5-Br_2$ | $-CH_2C_6H_5$ | $-CH_2C_6H_5$ | $-SO_2-$ | |
| 2-42 | $-CH_3$ | $3,5-(CH_3)_2$ | $3,5-(CH_3)_2$ | $3,5-(CH_3)_2$ | $3,5-(CH_3)_2$ | H | H | $-SO_2-$ | |
| 2-43 | $-CH_3$ | $3-CH_2CH=CH_2$ | $3-CH_2CH=CH_2$ | $3-CH_2CH=CH_2$ | $3-CH_2CH=CH_2$ | H | H | $-SO_2-$ | |
| 2-44 | $-CH_3$ | $3,5-Br_2$ | $3,5-Br_2$ | $3-CH_2CH=CH_2$ | $3-CH_2CH=CH_2$ | H | H | $-SO_2-$ | |
| 2-45 | $-CH_3$ | $3,5-Br_2$ | $3,5-Br_2$ | $3-CH_3$ | $3-CH_3$ | H | H | $-SO_2-$ | |
| 2-46 | $-CH_3$ | $3,5-Br_2$ | $3,5-Br_2$ | $3,5-(CH_3)_2$ | $3,5-(CH_3)_2$ | H | H | $-SO_2-$ | |
| 2-47 | $-CH_3$ | $3,5-Br_2$ | $3,5-Br_2$ | — | — | H | H | $-SO_2-$ | |
| 2-48 | $-CH_3$ | $3,5-Br_2$ | $3,5-Br_2$ | $3,5-Br_2$ | $3,5-Br_2$ | H | H | $-SO_2-$ | |
| 2-49 | H | — | — | — | — | $-CH_2C_6H_5$ | $-CH_2C_6H_5$ | -CO- | oily |
| 2-50 | H | — | — | — | — | $-CH_2C_6H_5$ | $-i-C_3H_7$ | -CO- | |
| 2-51 | H | — | — | — | — | $-i-C_3H_7$ | $-i-C_3H_7$ | -CO- | |
| 2-52 | H | $3-CH_3$ | $3-CH_3$ | — | — | $-CH_2C_6H_5$ | $-CH_2C_6H_5$ | -CO- | |
| 2-53 | H | $3-CH_3$ | $3-CH_3$ | $3-CH_3$ | $3-CH_3$ | $-CH_2C_6H_5$ | $-CH_2C_6H_5$ | -CO- | |
| 2-54 | H | $3-CH_2CH=CH_2$ | $3-CH_2CH=CH_2$ | — | — | $-CH_2C_6H_5$ | $-CH_2C_6H_5$ | -CO- | |
| 2-55 | H | $3-CH_2CH=CH_2$ | $3-CH_2CH=CH_2$ | $3-CH_2CH=CH_2$ | $3-CH_2CH=CH_2$ | $-CH_2C_6H_5$ | $-CH_2C_6H_5$ | -CO- | |
| 2-56 | H | $3,5-Br_2$ | $3,5-Br_2$ | — | — | $-i-C_3H_7$ | $-i-C_3H_7$ | -CO- | |
| 2-57 | H | — | — | — | — | H | H | -CO- | 114-117 |
| 2-58 | H | $3-CH_2CH=CH_2$ | $3-CH_2CH=CH_2$ | — | — | H | H | -CO- | |
| 2-59 | H | $3-CH_3$ | $3-CH_3$ | — | — | H | H | -CO- | |

According to commonly known manufacturing method, the derivatives of 2-propanol represented by general formula (I) is obtained without any problem.

For example, 2-propanol derivatives represented by the general formula (I) in which Y is general formula (II) can be prepared in following reaction:

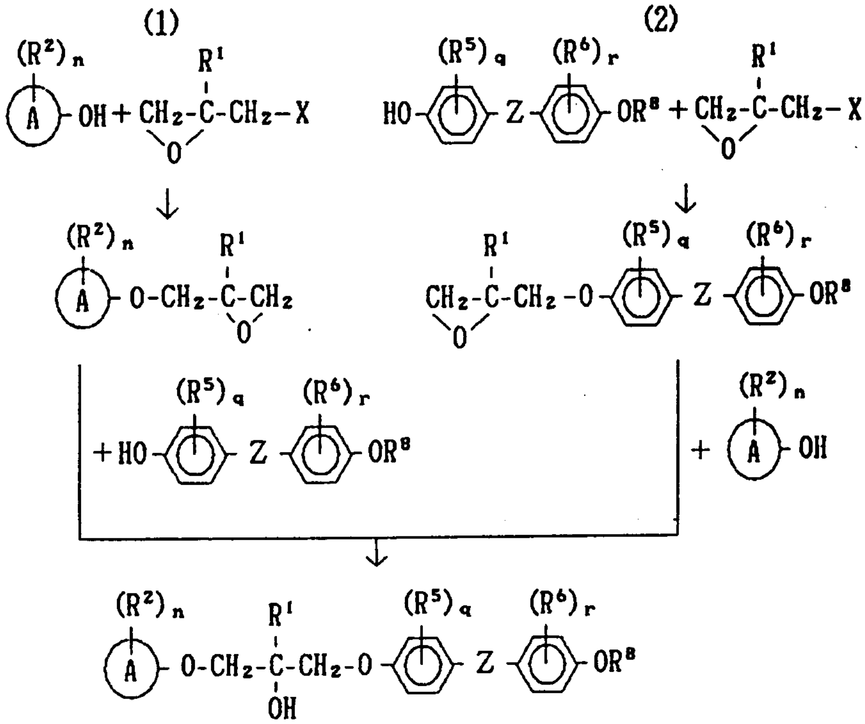

(Wherein, X is halogen and the other substituents are same as described above.)

Two methods are possible and the example in the case htat Z is $-SO_2-$ is explained. These methods are additionally described as follows:

In the method (1), arylhydroxy and epihalohydrin are reacted to synthesize arylglycidyl ether at the first step and then reacted with 4-hydroxy-4'-substituted diphenylsulfone derivatives.

In the method (2), 4-hydroxy-4'-substituted diphenylsulfone and epihalohydrin are reacted at the first step to synthesize 4-glycidyloxy-4'-substituted diphenylsulfone and then reacted with arylhydroxy derivative.

As described above, according to the following reaction formula, 2-propanol derivatives represented by general formula (I) in which Y is general formula (III) are also possible to be prepared.

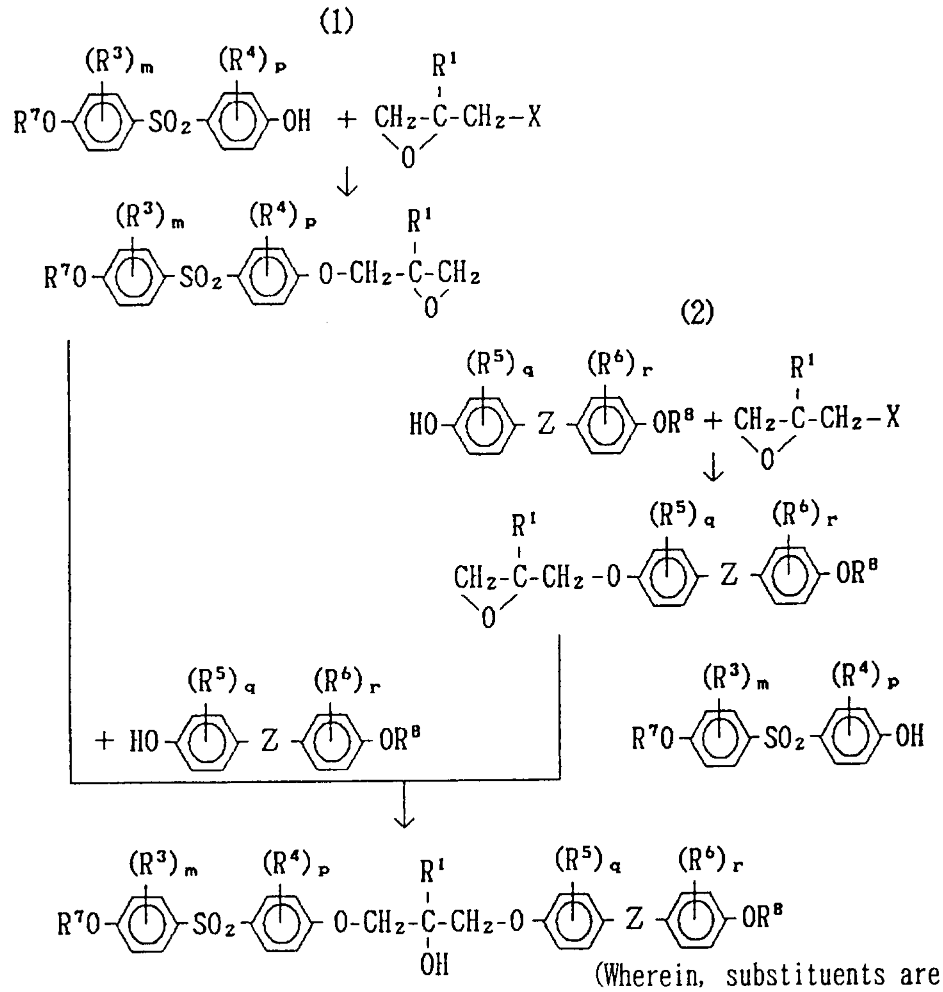


(Wherein, substituents are as above)

The reacting equation as described above, that is, the preparing methods for these compounds are practically described as follows:

In the second step reaction between a hydroxy compound and a glycidyl ether react, for example, the desired compound is obtained by their reaction by heating at 70 ~160 °C in non-protonic solvents such as dimethylformamide, dimethyl-acetamide, dimethylsulfoxide or sulforane, or aromatic solvent such as chlorobenzene, toluene or xylene under presence of organic base such as tetrabutyl ammonium bromide, triethylamine, 1,8-diazabicyclo-[5,4,0]-7-undecene or inorganic base such as sodium hydroxide or potassium hydroxide.

On the other hand, if $R^7$ and $R^8$ are hydrogen, the protective method is preferable to obtain the desired compound. Benzylation according to the usual manner is probable before the reaction to prevent formation of byproducts at the positions.

The compounds which are possible to be prepared by the methods as above, behave polymorphizmic crystalline property or to adduct with the solvent depending upon the crystalline deposition condition, for example, kind of solvent and their deposition temperature.

These difference can be confirmed by measurement of melting point and infra-red spectrometry, etc.

It is noteworthy that the invented compound is possible to apply not only for a storing image stabilizing agent further proofing property against plasticizer to prevent decolorization of the image, but also for a color developer or a sensitizing agent depending upon the substituents $R^7$ and $R^8$. Describing more substantially, in the case that $R^7$ and $R^8$ as substituents are hydrogen and the invented compound are hydroxylated nuclei compound, the derivatives further provide color developing function. Therefore, they can be applied for color developer with the excellent proofing property against plasticizer. The characteristics are outstanding in 2-propanol derivatives of the general formula (I) in which Y is general formula (II).

Contrarily, if the substituents are not hydrogen, the derivatives additionally provide sensitizing function and are possible to use for an excellent sensitizing agent with the proofing property against plasticizer.

According to common process, the recording material including the leuco chromogen can be prepared. For example, for a storing image stabilizing agent the invented compounds are used with the color developer, various sensitizers, adjuvants and for color developer with various sensitizers and adjuvants, and for sensitizers the invented compound is used with color developer. Of course, it is possible to manufacture commercially recording materials by combination of the invented compounds in which one acts as the storing image stabilizing agent and the other does as the color developer or as the sensitising agent. It is also possible to produce recording materials providing a specific function in the color development by combining the invented compound with any compound which is known to be a same purpose as the invented compound.

It is very beneficial that the invented compound acts multiple specific roles as a storing image stabilizing agent and a color developer or a sensitizing agent, since the recording materials are possible to be manufactured with low cost due to diminish relative charges in amount of the storing image stabilizing agent and a color developer or a sensitizing agent to a leuco chromagen in each other.

The invented compounds are applicable to any recording material in which any leuco chromogens are included. For example, the compounds is applicable to both purposes in thermal sensitive recording papers and pressure sensitive copying papers, etc.

When the invented compounds are applied to a thermal sensitive paper, they are allowed to be processed similarly as known storing image stabilizing agent, color developer or sensitizing agent. For example, the recording material can be manufactured by dispersing the invented compound and a chromogen in aqueous solution with a soluble binder. The suspension is coated on a holding material such as paper and is dried. The invented compound is allowed to be contained in coloring chromogen layer as described above. In the case that coated structure is consisted of multi-layer, the compound is allowed to be contained in an optional layer, for example, in a protective layer, etc. In this case, Applying the invented compound for a storing image stabilizing agent, ratio of the invented compound to a leuco chromogen is recommended to be 0.1 ~5 or more desirably 0.2 ~2 parts to one part in weight of a leuco chromogen. If the invented compound applied for a color developer or a sensitizing agent, it is recommended that the ratio is 1~10, more desirably 1.5~5 part in weight to one part in weight of a chromogen.

The other color developer, image stabilizing agent, sensitizing agent, filler, dispersing agent, antioxidant, antisticker, antifoaming agent, photo-stabilizing agent and fluorescent brightening agent, etc., may be additionally contained in the dispersion described above, if necessary.

【0022】

Derivatives of fluorane, phthalide, lactum, triphenyl methane, phenothiazine and spiropyrane, etc., can be demonstrated as example of the leuco chromogens for recording materials in the invention. However, the invention shall be restricted in those compounds. Any leuco chromogen which provides color developing property by contacting with acidic color developers is applicable.

Following examples of fluorane derivatives in the leuco chromogens can be demonstrated:

3-diethylamino-6-methyl-7-anilinofluorane,
3-dibutylamino-6-methyl-7-anilinofluorane,
3-(N-ethyl-N-isobutylamino-6-methyl-7-anilinofluorane,
3-(N-methyl-N-propylamino-6-methyl-7-anilinofluorane,
3-(N-ethyl-N-isopentylamino-6-methyl-7-anilinofluorane,
3-diethylamino-7-(o-chloroanilino)-fluorane,
3-dibutylamino-7-(o-chloroanilino)-fluorane,
3-diethylamino-7-dibenzylaminofluorane,
3-diethylamino-5-methyl-dibenzylaminofluorane,
3-(N-ethyl-p-toluidino)-6-methyl-7-anilinofluorane,
3-(N-cyclohexyl-N-methylamino)-6-methyl-7-anilinofluorane,
3-(N-ethyl-N-isobutylamino)-5,6-benzofluorane,
3-pyrrolydino-6-methyl-7-anilinofluorane and
3-pyperidino-6-methyl-7-anilinofluorane, etc.

When an invented compound is applied for a storing image stabilizing agent or for a color developer combining with the other color developer, the selection is optional in common colordevelopers used in thermal sensitive recording materials. The representative examples of color developer are described as follows:

Bisphenols such as Bisphenol A, 4,4'-sec-butylidene bisphenol, 4,4'-cyclohexylidene bisphenol, 2,2-dihydroxydiphenyl, pentamethylene-bis(4-hydroxybenzoate), sulfur containing bisphenols such as 1,7- (4-hydroxy-phenylthio)-3,5-dioxaheptane, hydroxy benzoic acid esters such as benzyl 4-hydroxybenzoate, ethyl 4-hydroxybenzoate, propyl 4-hydroxy benzoate, isopropyl 4-hydroxy benzoate, butyl 4-hydroxy benzoate, isobutyl 4-hydroxy benzoate, chlorobenzyl 4-hydroxybenzoate methylbenzyl 4-hydroxybenzoate, diphenylmethyl 4-hydroxybenzoate, metal salts of benzoic acid such as zinc benzoate. zinc 4-nitrobezoate, dihydroxydiphenyl-sulfones such as 4-hydroxy-4'-methyldiphenylsulfone, 4-hydroxy-4'-isopropoxydiphenylsulfone, 4-hydroxy-4' -b utoxydiphenylsulfone, 4-hydroxyphthalic acid diesters such as dimethyl 4-hydroxyphthalate, dicyclohexyl 4-hydroxy-phthalate, diphenyl 4-hydroxyphthalate, hydroxy naphthoic acid esters such as 2-hydroxy-6-carboxynaphthalene, hydroxyacetophenone, p-phenylphenol, benzyl 4-hydroxyphenylacetate, p-benzylphenol, hydoroquinone monobenzylether and tribromomethylsulfones such as tribromomethylphenylsulfone, etc.

When a compound is applied for a sensitizing agent combining with the other ones in thermal sensitive recording materials, the sensitizing agent can be optionally selected.

The typical examples of the sensitizing agent can be demonstrated as follows:

Higher fatty acid amides, benzamides, stearic anilide, acetoacetic anilide, thioacetanilide, dibenzyl oxalate, dimethylphthalate, dibenzyl telephthalate, dibenzyl isophthalate, bis-(tert-butylphenol) compounds, 4,4'-dihydroxydiphenylsulfone diethers, 1,2-bis(phenoxy) ethane, 1,2-bis(4-methylphenoxy) ethane, 1,2-bis-(3-methylphenoxy) ethane, 2-naphthol benzyl ether, diphenylamine, carbazole, 2,3-di-m-tolylbutane, 4-benzylbiphenyl, 4,4'-dimethyl-biphenyl, m-terphenyl and di-beta-naphthylphenylenediamine, etc..

For example in the filler, clay, talc, kaolinite, satinwhite, titanium oxide, calcium carbonate, magnesium carbonate, barium sulfate, magnesium silicate and aluminum silicate, etc., can be demonstrated.

For example in the dispersing agent, sulfosuccinic acidesters such as sodium dioctylsuccinate, sodium dodecylbenzene sulfonate, sodium lauryl alcohol sulfate and fatty acid salts, etc., can be demonstrated.

For example in storing image stabilizing agent, salicylicacid derivatives, metal salts of oxynaphthoic acid derivatives (especially, zinc salt) and the other insoluble zinc compounds, etc., can be demonstrated.

For example in antioxidant, 2,2'-methylenebis(4-methyl-6-tert- butylphenol), 2,2'-methylenebis (4-ethyl-6-tert-butylphenol), 4,4'-propyl-methylenebis (3-methyl-6-tert-butylphenol), and 4,4'-thiobis (2-tert-5-methylphenol),etc., can be demonstrated.

For example in sensitizing agent, aliphatic higher alcohols, polyethylene glycols and guanidine derivatives, etc., can be demonstrated.

For example in anti-sticker stearic acid, zinc stearate, calcium stearate, carnauba wax, paraffin wax and ester wax, etc., can be demonstrated.

When a pressure sensitive copying paper is manufactured from the invented compound. similar methods as known storing image stabilizing agent, color developer or sensitizing agent, can be applied. For example, a leuco chromogen micro-capsulized with proper dispersing agent is dispersed according to known method and is coated on paper to prepare chromogenic sheet. The dispersion liquid for color developer is also coated on paper to prepare color developer sheet. In the case described above when the invented compound is applied for a storing image stabilizing agent, the compound is used by adding in the dispersion liquid for either coloring sheet or the color developing sheet.

Thus, the pressure sensitive copying papers are manufactured by combining both sheets. The pressure sensitive copying papers can be made by a unit made of an upper layer paper which is coated and impregnated with microcapsule enveloping the leuco chromogen solution in organic solvent at lower surface and a lower layer paper which is coated and impregnated with color developer at upper surface. Additionally so called "self content paper" that is coated at a same surface with the microcapsule and the color developer is allowable.

Best Mode to Execute the Invention:

The invention is described in detail, giving actual examples as follows. However the invention shall be never restricted by the following description.

Example 1. (Synthesis of compound 1-1 in Table 1)

In 100 ml of dimethylformamide, 15 g of phenyl glycidyl ether and 37.5 g of 4,4'-dihydroxydiphenylsulfone were dissolved by heating. Further 0.2 g of 1,8-diazabicyclo[5, 4,0]-7-undecene was added in the solution. The mixture was reacted at 100 °C with stirring for 3 hours. The reacted product was dispersed into ice water and extracted by ethyl acetate. Organic layer separated was washed by saturated sodium carbonate aqueous solution, then was dehydrated by anhydrous sodium sulfate.

The solvent was removed by distillation. The formed crude substance was recrystallized from ethyl acetate solution. Thus, 25 g of white crystalline 1-phenoxy-3-(4-hydroxy-diphenylsulfon-4'-yloxy)-2-propanol with melting point 145 ~148 °C was obtained. The chemical formula was collectively shown in Fig. 1.

Phenyl glycidyl ether used as raw material is synthesized as follows:

In 500 ml of 10 % sodium hydroxide 2 g of benzyl tri-n-butyl ammonium chloride and 94 g of phenol were dissolved and the solution was cooled to room temperature. Then. 92.5 g of epichlorohydrin was added and the solution was stirred for 2 hours. Separated oil layer from the solution was extracted by diethyl ether. The extract was washed by water, dehydrated to dry and the solvent was removed by distillation. The residual material was distilled under reduced pressure. Thus, 105 g of refined colorless liquid phenyl glycidyl ether was obtained from the distillate fraction with 126~128 °C/17 mm Hg.

Example 2. (Synthesis of compound I- 10 in Table 1)

According to similar synthetic method as phenyl glycidyl ether in example 1, 4-benzyloxy-4'-glycidyloxydiphenyl-sulfone was obtained by reacting 4-benzyloxy-4'-hydroxydiphenylsulfone and epichlorohydrin in sodium hydroxide aqueous solution. In 10ml of dimethylformamide, 5 g of the obtained compound was dissolved. In the solution. 1.5 g of p-cresol and 1,8-diazabicyclo[5,4,0]-7-undecene were further added.

The mixture was reacted by heating and refluxing for 2 hours and was dispersed into water after the reaction. The dispersion was extracted by ethyl acetate. The organic layer was washed by 4 % aqueous solution of sodium hydroxide. The solvent was removed by distillation. The residual material was heated to refluxd with 15 ml of hydrochloric acid and 15 ml acetic acid for 2 hours.

The reacted material with adding water extracted by chloroform. Then, the extract was refined through silica gel chromatography (mobile phase, chloroform:methanol = 95:9) and recrystallized further from ethyl acetate solution. Thus, 4.1 g of white crystalline 1-(4-methylphenoxy)-3-(4-hydroxydiphenylsulfon-4'-yloxy)-2-propanol with melting point 170~ 172 °C was obtained.

Example 3. (Synthesis of compound I- 56 in Table 1)

According to similar synthetic method as phenyl glycidyl ether in example 1, 4-benzyloxy-4'-(2-methylglycidyloxy) diphenylsulfone was obtained by reacting with 4-benzyloxy-4'-hydroxydiphenylsulfone and 2-methylepichlorohydrin in sodium hydroxide aqueous solution. Five grams of the compound and 2.3 g of p-phenylphenol were similarly reacted as example 2. Thus, 4.2 g of crystalline 1-(4-phenylphenoxy)-3-(4-hydroxydiphenyl-sulfon-4'-yloxy)-2-methyl-2-propanol with melting point 163 ~165 °C was obtained. The chemical formula was collectively shown in Fig. 1.

Example 4. (Synthesis of compound I- 66 in Table 1)

In example 1, instead of 37.5 g of 4,4'-dihydroxy diphenyl sulfone and 100 ml of dimethylformamide as a solvent, 41 g of2,2-bis (4-hydroxyphenyl) propane and 90 ml of methyl isobytyl ketone were used, respectively. Ten grams of crystalline 2-(4-(2-hydroxy-3-phenoxy propoxy) phenyl)-2-(4-hydroxyphenyl)-propane with melting point 128-131 °C was obtained according to similar procedures as example 1.

Example 5. (Synthesis of compound I- 85 in Table 1)

In 30 ml of methyl isobutyl ketone, 12 g of 4-benzyloxy-4'-(2-methylglycidyloxy) benzophenone and 3 g of phenol were added. Furthermore, 0.5 g of 1,8-diazabicyclo[5, 4, 0]-7-undecene was also added. Then, they were reacted by heating to refluxing for 3 hours. After the reaction, recovering the solvent under reduced pressure. 15 g of crude 1-phenoxy-3-(4-benzyloxybenzo-phenon-4'-yloxy)-2-methyl-2-propanol was obtained.

Twenty five ml of acetic acid and 25ml of concentrated hydrochloric acid were added to the crude material and the mixture was reacted by heating to refluxing for 3 hours. The reacted liquid was poured into 200 ml of methyl isobutyl ketone. After separating and washing the mixture by water, it was further washed by sodium bicarbonate aqueous solution. The desired compound was extracted by adding 5 ml of 8 % sodium hydroxide aqueous solution to remove organic solvent with separatory funnel procedure. Adding hydrochloric acid in the alkaline aqueous layer to be acidic, the desired substance was deposited. Filtering and recrystallizing from ethyl acetate solution. 7 g of crystalline 1-phenoxy-3-(4-hydroxybenzophenon-4-yloxy)-2-methyl-2-propanol with melting point 155~162 °C was obtained.

The chemical equation was collectively shown in Fig. 1.

This synthetic method is an example to protect the hydroxyl with benzyl group. The starting material, 4-benzyloxy-4'-(2-methylglycidyloxy) benzophenone, was synthesized as follows:

In 1% sodium hydroxide aqueous solution, 21.4 g of benzophenone was dissolved by heating and benzyl chloride was slowly dropped into the solution at 70 °C taking for 2 hours. The mixture was continued to heat and to react with stirring more 3 hours. Deposited crystal was filtered and washed by water. Thus, 26 g of 4-hydroxy-4'-benzyloxybenzophenone was obtained.

Additionally, 50 ml of 2-methylepichlorohydrin and 1 ml of triethanolamine as a catalyst were added to 25 g of 4-hydroxy-4'-benzyloxybenzophenone and the mixture was reacted at 80 °C for 6 hours. Completing the reaction excess quantity of 2-methyl epichlorohydrin was removed by distillation under reduced pressure. In the residual material 200 ml of toluene was added to dissolve it by heating. The solution was reacted to dehydrate azeotropically by addition with dropping 10 g of 40 % sodium hydroxide. Them, toluene was recovered and the desired compound was recrystallized from toluene solution. Thus, 31 g of crystalline 4-benzyloxy-4'-(2-methyl-glycidyloxy) benzophenone was obtained.

Structures of the compounds synthesized in example 1-5 were shown in Table 3 for taking a notice.

Table 3

| | | |
|---|---|---|
| Example 1. | compound 1-1 | 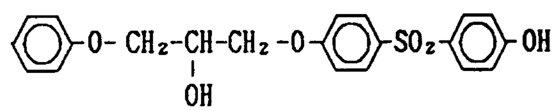 |
| Example 2. | compound 1-10 | 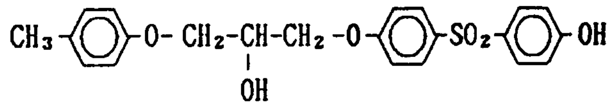 |
| Example 3. | compound 1-56 | 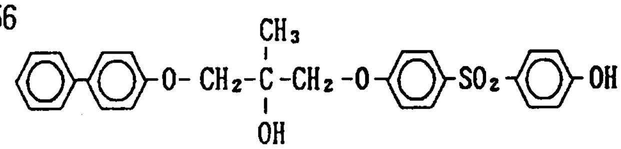 |
| Example 4. | compound 1-66 | 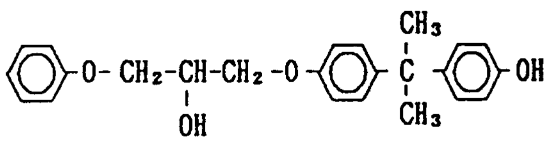 |
| Example 5. | compound 1-85 | 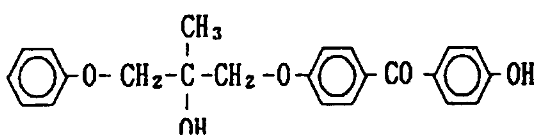 |

Compounds in which Y is general formula (II) in the general formula (I), synthesized in example 1, 2, 3, 4 and 5 described above and also done by modified method according to these examples, were collectively shown in Table 1 with the actual examples. In additional comment to the table, term expressed as "oily" indicates that condition of the obtained compound was hardly crystallized and impossible to measure its melting point.

Example 6. (Synthesis of compound 2-25 in Table 2)

In 150 ml of dimethylformamide, 20.5 g of 4-benzyloxy-4'-(2-methyl-glycidyloxy) diphenylsulfone prepared in example 3, 17 g of 4-benzyloxy-4'-hydroxydiphenylsulfone and 5 ml of triethylamine were added. The solution was reacted at 150 °C for 6 hours. After the reaction, dispersing the solution in 500 ml of water, the crude material was obtained. After washing the material successively by hot ethanol and hot toluene with this order, 13 g of crystalline 1,3-bis-(4-benzyloxydiphenylsulfon-4'-yloxy)-2-methyl-2-propanol with melting point 204~205 °C was obtained.

Example 7. (Synthesis of compound 2-27 in Table 2)

In 300 ml of toluene, 41 g of 4-benzyloxy-4'-(2-methyl-glycidyloxy)diphenylsulfone prepared in example 3, 28 g of 4-isopropoxy-4'-hydroxydiphenylsulfone and 5 ml of triethyamine were added. The solution was reacted with refluxing for 6 hours. Cooling the reacted solution, deposited crystal was filtered. Thus, 34 g of crystalline 1-(4-benzyloxydiphenylsulfon-4'yloxy)-3-(4-isopropyloxydiphenylsulfon-4'-yloxy)-2-methyl-2-propanol with melting point 217 ~220 °C was obtained.

Example 8. (Synthesis of compound 2-24 in Table 2)

Reacting 4-benzyloxy-4'-glycidyloxydiphenyl-sulfone prepared in example 2 and 4-benzyloxy-4'-hydroxydiphenylsulfone according to example 6, crystalline 1,3-bis-(4-benzyloxy-diphenylsulfon-4'-yloxy)-2-propanol [compound 2-1 in Table 2] with melting point 201-205 C was obtained. Fifty ml of hydrochloric acid and 50 ml of acetic acid were added to 27 g of the compound. The mixture was heated to disappear insoluble matter with refluxing for about 5 hours. The reacted solution was poured in 200 ml of methyl isobutyl ketone and the mixture was extracted by the solvent. Separating in a funnel and washing with water the solvent was removed to recover. Recrystallizing to refine the crude material obtained by 5 % methanol/chloroform solvent. 9.5 g of crystalline 1,3-bis(4-hydroxydiphenylsulfon-4'-yloxy)-2-propanol was obtained with melting point 168~170 °C.

Example 9. (Synthesis of compound 2-57 in Table 2)

In 100 ml of methyl isobutyl ketone. 10 g of 4-benzyloxy- 4'-glycidyloxydiphenylsulfone prepared in example 2 and 9 g of 4-benzyloxy-4'-hydroxydiphenylsulfone were dissolved followed by adding 1 ml of triethylamine. The solution was heated to reflux for 5 hours. After washing the reacted solution by 10 % sodium hydroxide aqueous solution, distilling to remove methyl isobutyl ketone and distillation under reduced pressure, 1-(4-bezyloxydiphenylsulfon-4'-yloxy)-2-propanol as an intermediate [compound 2-49 in Table 2] was obtained.

In the next step 30 ml of acetic acid and 30 ml concentrated hydrochloric acid were added to the obtained compound. After the mixture was refluxed by heating for 5 hours. the reacted liquid was poured into 200 ml of methyl isobutyl ketone to extract. After separating the organic layer with a funnel, washing by water and removing the solvent to recovery, the crude desired material was obtained. By recrystallizing it from 5 % methanol/ chloroform solution to refine, 7 g of crystalline 1-(4-hydroxydiphenylsulfon-4'-yloxy)-3-(4-hydroxybenzophenon-4'-yloxy)-2-propanol with melting point 114 ~117 °C was obtained.

Structures of the compounds synthesized in example 6~9 were shown in Table 4 for taking a notice.

Table 4.

Example 6. compound 2-25

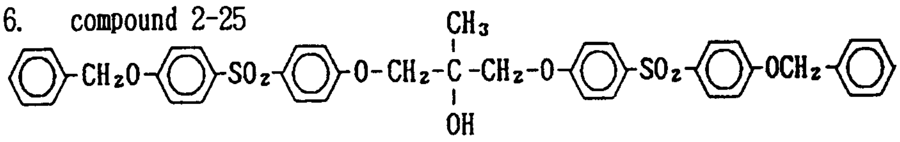

Example 7. compound 2-27

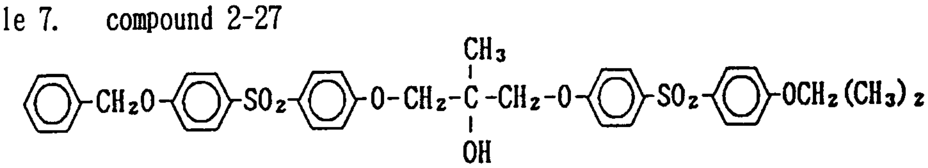

Example 8. compound 2-24

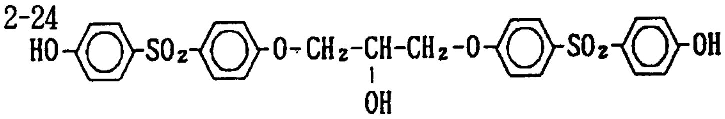

Example 9. compound 2-57

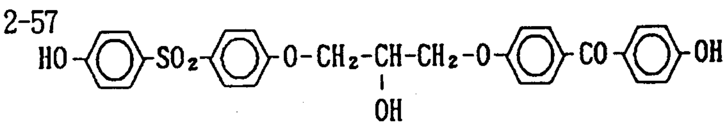

Compounds in which Y is general formula (III) in the general formula (I), synthesized in example 5, 6, 7, 8 and 9 described above and also done by modified method according to these examples, were collectively shown in Table 2 with the actual examples. In additional comment to the table, term expressed as "oily" indicates that condition of the obtained compound was hardly crystallized and impossible to measure its melting point.

Example 10 (Preparation of thermal Sensitive Recording Paper)

| Chromogen dispersion (Liquid A) | |
|---|---|
| 3-dibutylamino-6-methyl-7-anilinofluorane | 7.0 g |
| 15 % polyvinyl alcohol aqueous solution | 41.5 g |
| Clay | 11.5 g |
| Purified water | 40.0 g |

| Color developer dispersion (Liquid B-1) | |
|---|---|
| 4-isopropoxy-4'-hydroxydiphenylsulfone | 10.5 g |
| 15 % polyvinyl alcohol aqueous solution | 41.5 g |
| Clay | 8.0 g |
| Purified water | 40.0 g |

| Color developer dispersion (Liquid B-2) | |
|---|---|
| 2,2-bis(4-hydroxyydiphenyl)propane | 7.0 g |
| 15 % polyvinyl alcohol aqueous solution | 41.5 g |
| Clay | 11.5 g |
| Purified water | 40.0 g |

| The invented compound dispersion (Liquid C) | |
|---|---|
| The invented compound | 7.0 g |
| 15 % polyvinyl alcohol aqueous solution | 41.5 g |
| Clay | 11.5 g |
| Purified water | 40.0 g |

| Clay dispersion (Liquid D) | |
|---|---|
| Clay | 18.5 g |
| 15 % polyvinyl alcohol aqueous solution | 41.5 g |
| Purified water | 40.0 g |

Each mixture of the composition described above was sufficiently homogenized by a sand grinder to prepare Liquid A, Liquid B-1, Liquid B-2, Liquid C and Liquid D. One weight part of Liquid A, 2 weight parts of Liquid B-1 or B-2 and one weight part of Liquid C was mixed to prepare two kinds of different coating liquids in color developer.

For a notice the ratio of chromogen to color developer in the coating liquids was that chromogen: color developer = 1 : 3 in Liquid B-1 and was that chromogen : color developer = 1 : 2 in Liquid B-2. After the liquid was coated by using Wire Rod (No. 12) the surface was dried. The coated paper was treated by Calendaring machine to smoothing the surface.

Thus, two kinds of thermal sensitive recording papers of the different mixing ratio as describe above, which were called as thermal sensitive recording paper-1 group in the former and sensitive recording paper-2 group in the latter were prepared.

These thermal sensitive recording papers are examples which the invented compounds are applied for storing image stabilizing agent.

Comparative example 1

In the case applying Liquid B-1 for color developer dispersion D instead of Liquid C in example 10, and the other components were same as example 10, the thermal sensitive recording paper-1 group containing no compound in the invention was prepared.

Comparative example 2

In the case applying Liquid B-2 for color developer, dispersion D instead of Liquid C in example 10, and the other components were same as example 10, the thermal sensitive recording paper-2 group containing no compound in the invention was prepared.

Example 11 (Tests for proofing property of the thermal sensitive recording paper against plasticizer)

The thermal sensitive recording paper-1 group and the paper-2 group prepared in example 10, comparative example 1 and 2 were colored to be mosaic pattern under the condition of 22 volts of impressed printing voltage and 1.8 ms of pulse width by Test Apparatus for Color Development of Thermal Sensitive Paper (Ohkura Denki Co., LTD. Type TH-PMD). In the state that a wrapping film made of polyvinyl chloride was closely touched on the colored surface of the tested recording papers as above, the proofing property against plasticizer was tested under following conditions.

In the case of compounds in which Y is general formula (II) in the general formula (I),

thermal sensitive recording paper-1 group was kept under the atmosphere at about 40 °C for 24 hours and 30 hours;

thermal sensitive recording paper-2 group was kept under the atmosphere at about 40 °C for 4 hours and 8 hours.

In the case of compounds in which Y is general formula (III) in the general formula (I),

thermal sensitive recording paper-1 group was stood still under the atmosphere at about 40 °C for 18 hours and 24 hours:

thermal sensitive recording paper-2 group was stood still under the atmosphere at about 40 °C for 4

hours and 8 hours.

Color density values before and after exposing as above treatment for this test were determined by Macbeth Reflection densitometer (RD-514, Filter: #106). Results. concerning to the compounds in which Y is general formula (II) and to the compounds in which Y is general formula (III) were shown in Table 5 and 6, respectively.

Table 5

| Thermal Sensitive Recording paper Group | Before Treatment Measured Value | After Treatment Measured Value | After Treatment Remaining Ratio | After Treatment Measured Value | After Treatment Remaining Ratio |
|---|---|---|---|---|---|
| Thermal Sensitive Recording Paper-1 | | 24 hrs. | | 30 hrs | |
| Compound 1-1 | 1.35 | 1.02 | 75.5 % | 0.86 | 63.7 % |
| Compound 1-10 | 1.27 | 0.75 | 59.1 % | 0.69 | 54.3 % |
| Compound 1-14 | 1.28 | 0.82 | 64.1 % | 0.58 | 45.3 % |
| Comparative Example 1 | 1.25 | 0.35 | 28.0 % | 0.28 | 22.4 % |
| Thermal Sensitive Recording Paper-2 | | 4 hrs. | | 8 hrs. | |
| Compound 1-3 | 1.24 | 0.46 | 37.1 % | 0.33 | 26.6 % |
| Compound 1-78 | 1.29 | 0.65 | 50.4 % | 0.55 | 42.6 % |
| Compound 1-90 | 1.28 | 0.52 | 40.6 % | 0.36 | 28.1 % |
| Compound 1-96 | 1.29 | 0.50 | 38.8 % | 0.33 | 25.6 % |
| Comparative Example 2 | 1.26 | 0.43 | 34.1 % | 0.30 | 23.8 % |

Table 6

| Thermal Sensitive Recording Paper Group | Before Treatment Measured Value | After Treatment Measured Value | Remaining Ratio | Measured Value | Remaining Ratio |
|---|---|---|---|---|---|
| Thermal Sensitive Recording Paper-1 | | 18 hrs. | | 24 hrs | |
| Compound 2-1 | 1.22 | 0.81 | 66.4 % | 0.76 | 62.3 % |
| Compound 2-25 | 1.20 | 0.71 | 59.1 % | 0.69 | 57.5 % |
| Comparative Example 1 | 1.20 | 0.38 | 31.7 % | 0.36 | 30.0 % |
| Thermal Sensitive Recording Paper-2 | | 4 hrs. | | 8 hrs. | |
| Compound 2-24 | 1.19 | 1.05 | 88.2 % | 0.95 | 79.8 % |
| Compound 2-57 | 1.15 | 1.02 | 88.7 % | 0.88 | 76.5 % |
| Comparative Example 2 | 1.18 | 0.43 | 36.4 % | 0.28 | 23.7 % |

Values shown in above table are the larger, the higher color density was indicated. The larger remaining rate indicates the less decoloring. Color density value determined after the exposing is divided by that of before exposing to calculate percentage of the remained ratio. The results validate that thermal sensitive recording paper using the invented compounds with color developers is excellent in the proofing property against plasticizers.

Example 12 (Preparation of a thermal sensitive paper containing a compound in Table 1 as color developer)

Using dispersions in example 10, a coating liquid composed of one weight part of Liquid A and three parts of Liquid C was prepared. A thermal sensitive paper was provided by same procedure as described in example 10. This preparation of thermal sensitive paper is an example in the case using a compound, in which $R^8$ is hydrogen atom in addition in which y is general formula (II) in the general formula (I), as a color developer.

Comparative example 3

In example, instead of the invented compound in Liquid C of example 10, a dispersion containing a resemble compound, 1-phenoxy-3-(4-hydroxyphenylthio)-2-propanol, was provided and a thermal sensitive recording paper was obtained by similar process as in example 12.

Example 13 (Test for thermal sensitive recording paper using a compound in Table 1 as a color developer)

The thermal sensitive recording papers prepared according to both examples 12 and comparative example 3 were colored to be mosaic pattern, respectively and their proofing property against plasticizer was tested as described in example 11. The test results were summarized in Table 7 in which the test results of proofing property against plasticizer in comparative example 1 were shown to parallel comparison. Moreover, A part of the thermal sensitive recording papers prepared in example 12 and comparative example 1 were heated to develop color satisfied at 150 °C by dry heating tester (Kishino Science Machinery Co.). Stability to humidity, heat and light (exposing at 80 % relative humidity, at 50 °C for 24 hours, exposing to carbon arc for 4 hours) of back ground and color image was tested by Fedemeter.

The results were shown in Table 8.

Table 7

| Kind of Color Developer in Thermal Sensitive Recording Paper | Proofness to Plasticizer | | | | |
|---|---|---|---|---|---|
| | Before Treatment | 24 hrs. | | 48 hrs. | |
| | Measured Value | Measured Value | Remaining Ratio | Measured Value | Remaining Ratio |
| Compound 1-1 | 1.21 | 0.82 | 66.4 % | 0.76 | 62.8 % |
| Compound 1-10 | 1.20 | 0.68 | 56.7 % | 0.52 | 43.3 % |
| Compound 1-14 | 1.18 | 0.87 | 73.7 % | 0.74 | 62.7 % |
| Comparative Example 1 | 1.25 | 0.35 | 28.0 % | 0.28 | 22.4 % |
| Comparative Example 1 | 1.24 | 0.13 | 10.5 % | 0.10 | 8.1 % |

It is obviously shown from Table 7 that the invented compounds are more excellent than both 4-hydroxy-4'-isopropoxydiphenyl sulfone and 1-phenoxy-3-(4-hydroxyphenylthio)-2-propanol in comparative example 1 and 3, respectively, when they were used alone to proof against plasticizer of thermal sensitive recording paper.

Table 8

| Kind of Color Developer in Thermal Sensitive Paper | Back Ground | | | Colored Image | | |
|---|---|---|---|---|---|---|
| | Before Treatment | Humidity Proof | Photo stability | Before Treatment | Humidity Proof | Photo Stability |
| Compound 1-1 | 0.08 | 0.08 | 0.13 | 1.26 | 1.20 | 1.13 |
| Compound 1-10 | 0.05 | 0.06 | 0.11 | 1.24 | 1.16 | 1.23 |
| Compound 1-14 | 0.06 | 0.07 | 0.13 | 1.24 | 1.24 | 1.24 |
| Comparative Example 1 | 0.09 | 0.08 | 0.15 | 1.31 | 1.30 | 1.24 |

The characteristics of the color developers were compared in Table 8. It is evidently shown from the table that the invented compounds are never inferior to commercially available one, 4-isopropoxy-4'-hydroxydiphenylsulfone, or superior to them in respect to the both functions tested for protection from coloring of the background and photo-stability of the colored image.

Example 14 (Test of thermal sensitive recording paper applying compound in Table 1 as a sensitizing agent)

Thermal sensitive recording papers containing the compound 2-25 and the compound in comparative example 1 were prepared according to example 10. Each sample was divided in two groups to test coloring sensitivity in two methods. Each one was tested by dynamic color development at 1.10 m sec. pulse width as Test example 1.

The other one was tested by static color development method at 105 ° C sing dry heating tester (Kishino Science Machinery Co., Type E-3). These conditions were adjusted to be nearly one in measured value of Macbeth Reflection Densitometer(RD-514).

The results were shown below.

| Thermal Sensitive Recording Paper | Dynamic Color development density | Static Color Development density |
|---|---|---|
| Compound 2-25 | 1.15 | 1.05 |
| Comparative Example 1 | 1.04 | 1.03 |

It was indicated that the invented compound exhibited to be sensitized insignificantly in the static color development test. Whereas it was found to be sensitized significantly in the dynamic coloring sensitivity.

Industrial Applicability.

The invention is concerning to novel compounds which improve storing image stability of the developed image and especially proofing property against plasticizer and to thermal sensitive recording materials applying them. For example, the materials applying them in thermal sensitive recording such as label, keep very stable coloring image, even under condition contacting with package materials made of polymers.
In addition, as they provide comparable functions developing color or sensitizing dynamic color development with similarly commercial products so far, the compounds are useful as outstanding color developer and sensitizing agent.

## Claims

1. Propanol derivatives represented by general formula (I).

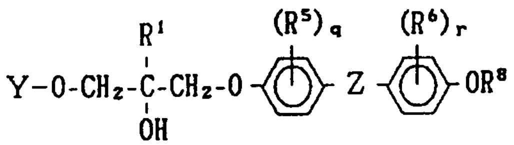

(I)

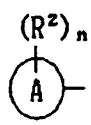

(II)

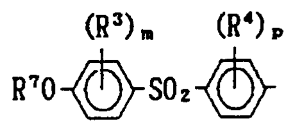

(III)

( herein: Y is a group represented by general formula (II) or (III) ,
$R^1$ is hydrogen or lower alkyl.
$R^2$ is halogen, nitro, lower alkyl, lower alkoxy, lower alkylthio, lower alkylsulfonyl, aryl, aryloxy, arylthio or arylsulfonyl, in which aryl may have substituent(s),
each of $R^3$, $R^4$, $R^5$ and $R^6$ is independently halogen, lower alkyl, lower alkoxy, lower alkenyl or lower alkenyloxy.
each of $R^7$ and $R^8$ is independently hydrogen, lower alkyl, lower alkenyl or aralkyl,
Z is bivalent lower alkyl, $-SO_2-$, -CO-, -S- or -O-,
ring A is benzene or naphthalene ring and
n, m, p, q and r denote an integral number from 0 through 4,
however, if n, m, p, q or r is 2 or more, $R^2$, $R^3$, $R^4$, $R^5$ or $R^6$ may be different. respectively, in each substituent.

2. Derivatives of 2-propanol represented by the general formula (I), wherein, the substituent Z is $SO_2$ or -CO-, described in claim 1.

3. Recording material applying a leuco chromogen that is characterized by containing one kind or more of 2-propanol derivatives for storing image stabilizing agent represented by general formula (I) as described in claim 1.

4. Recording material applying a leuco chromogen that is characterized by containing one kind or more of 2-propanol derivatives in which $R^8$ is hydrogen in the general formula (1) [wherein, Y is represented by the general formula (II)] for color developer as described in claim 1.

# INTERNATIONAL SEARCH REPORT

International Application No PCT/JP92/01206

**I. CLASSIFICATION OF SUBJECT MATTER** (if several classification symbols apply, indicate all) [6]

According to International Patent Classification (IPC) or to both National Classification and IPC Int. $Cl^5$

C07C43/23, 43/295, 49/84, 205/37, 317/22, 323/20, 323/21, C09B11/00, 57/00, 62/00, B41M5/26

**II. FIELDS SEARCHED**

Minimum Documentation Searched [7]

| Classification System | Classification Symbols |
|---|---|
| IPC | C07C43/20-43/23, 43/257-43/295, 49/84, 205/35-205/38, 317/16-317/22, 323/18-323/21, C09B11/00-11/28, 57/00, 62/00, B41M5/26 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | Chemical Abstracts, Vol. 83, No. 2, July 14, 1975 (14. 07. 75), (Columbus. Ohio. USA), N. N. Khromova, M. F. Sorokin, "Reaction of hydroxyalkyl ethers of dihydroxydiphenylpropane with epichlorohydrin", refer to Abstract No. 10916f, Tr. Mosk. khim.-Tekhnol. Inst.,1973 (74), p. 90-91 | 1 |
| X | Chemical Abstracts, Vol. 79, No. 3, July 23, 1973 (23. 07. 73), (Columbus. Ohio. USA), N. N. Khromova, M. F. Sorokin, "Synthesis of substituted hydroxyalkyl ethers of dihydroxydiphenylpropane", refer to Abstract No. 18272f, Tr. Mosk. Khim.-Tekhnol. Inst., 1972 (70), p. 71-73 | 1 |
| A | Chemical Abstracts, Vol. 79, No. 3, July 23, 1973 (23. 07. 73), (Columbus. Ohio. USA), | 2 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 25, 1992 (25. 11. 92) | December 15, 1992 (15. 12. 92) |
| **International Searching Authority** | **Signature of Authorized Officer** |
| Japanese Patent Office | |